# EUROPÄISCHE PATENTSCHRIFT

(11) **EP 0 816 325 B1**
(45) Veröffentlichungstag und Bekanntmachung des Hinweises auf die Patenterteilung: **05.04.2000**
(21) Anmeldenummer: 97110254.6
(22) Anmeldetag: 23.06.1997
(51) Int. Cl.: C07C 219/06, C07C 69/30, D06M 13/224

(54) **Kondensationsprodukte von Fettsäuren mit Aminoalkoholen und Polyolen und ihre Verwendung als Textilhilfsmittel**
Condensation products from fatty acids with aminoalcohols and polyols and their use as textile auxiliaries
Produits de condensation à partir d'acides gras avec aminoalcohols et polyols et leur utilisation comme produits auxiliaires textiles

(30) Priorität: 01.07.1996 DE 19626317
(43) Veröffentlichungstag der Anmeldung: 07.01.1998
(73) Patentinhaber: BASF AKTIENGESELLSCHAFT, 67056 Ludwigshafen (DE)
(72) Erfinder: Demirci, Faruk, 67434 Neustadt (DE); Simenc, Toni, 68199 Mannheim (DE)

(56) Entgegenhaltungen:
- EP-A- 0 415 279
- DD-A- 287 742
- DE-A- 3 730 792

## Beschreibung

Die vorliegende Erfindung betrifft neue Kondensationsprodukte von Fettsäuren mit Aminoalkoholen und Polyolen, ein Verfahren zur Herstellung dieser Kondensationsprodukte sowie deren Verwendung als Textilhilfsmittel, insbesondere als Behandlungsmittel zur Verminderung der Vergilbung beim Lagern von gefärbten Textilien und als Weichmacher für die vergilbungsarme Hochveredlung von Textilien.

Indigofärbungen können beim Lagern der fertigen Bekleidungsstücke (Denimartikel), beispielsweise in Verkaufsräumen oder im Freien vor Geschäften oder auf Märkten, durch Umgebungseinflüsse wie Licht, Sauerstoff, Ozon, Stickoxide oder Hitze an den exponierten Teilen vergilben (Lagergilbe). Diese Kleidungsstücke können dadurch schwer oder gar nicht mehr verkauft werden. Vor allem im Sommer führt diese Erscheinung zu häufigen Reklamationen und dadurch zu finanziellen Verlusten.

Aus der EP-B 415 279 sind Textilhilfsmittel bekannt, die bei der Herstellung von Textilien eingesetzt werden und sich durch Unterdrückung der Vergilbungsneigung und gute weichmachende Wirkung auszeichnen. Die dort beschriebenen Produkte sind jedoch Umsetzungsprodukte von Fettsäuremonoethanolamiden mit Ethylenoxid, besitzen also eine vollkommen andere chemische Struktur.

Aufgabe der vorliegenden Erfindung war es daher, ein Mittel bereitzustellen, das die Vergilbung beim Lagern von gefärbten Textilien, insbesondere von Indigofärbungen, vermindert. Außerdem sollte dieses Mittel den hiermit behandelten Textilien einen weichen Warengriff verleihen.

Demgemäß wurden Kondensationsprodukte von Fettsäuren der allgemeinen Formel I 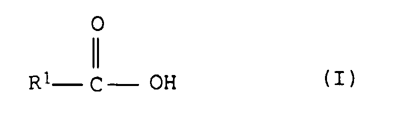 in der
- R¹: einen C₉- bis C₂₁-Alkyl- oder -Alkenylrest bedeutet,
mit 5 bis 150 mol-% pro Mol I Aminoalkoholen der allgemeinen Formel II 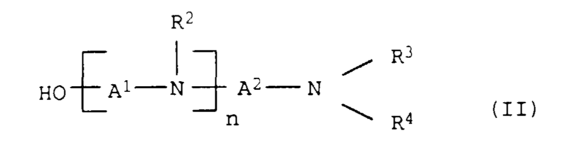 in der
- A¹ und A²: C₂- bis C₄-Alkylengruppen bezeichnen,
- R², R³ und R⁴: jeweils Wasserstoff, C₁- bis C₄-Alkyl oder eine Gruppierung -A¹-OH bzw. -A²-OH bedeuten und
- n: für eine ganze Zahl von 1 bis 5 steht,
und 5 bis 200 mol-% pro Mol I Polyolen der allgemeinen Formel III

HO―CH₂―X―CH₂―OH (III)

in der
- X: für eine chemische Bindung oder eine Gruppierung -CR⁵R⁶- steht, wobei
- R⁵: Wasserstoff, C₁- bis C₄-Alkyl, eine weitere Hydroxylgruppe oder eine Hydroxyalkylgruppe mit 1 bis 4 C-Atomen bezeichnet und
- R⁶: Wasserstoff, C₁- bis C₄-Alkyl oder eine Hydroxyalkylgruppe mit 1 bis 4 C-Atomen bedeutet,
gefunden.

In einer bevorzugten Ausführungsform werden die Fettsäuren I mit 40 bis 70 mol-% pro Mol I Aminoalkoholen II und 30 bis 120 mol-% pro Mol I Polyolen III kondensiert. Dabei kann es von Vorteil sein, daß die Summe aus den Komponenten II und III einen stöchiometrischen Überschuß bildet, im resultierenden Kondensationsprodukt gegebenenfalls noch unumgesetzt vorliegende Restmengen an II und/oder III stören nicht oder können sogar erwünscht sein.

Der Rest R¹ steht vorzugsweise für verzweigtes oder vor allem lineares C₁₁- bis C₁₉-Alkyl- oder -Alkenyl und insbesondere für C₁₃- bis C₁₇-Alkyl- oder -Alkenyl. Die Gruppierung R¹-CO- leitet sich beispielsweise von der Laurinsäure, Myristinsäure, Elaidinsäure, Linolsäure, Linolensäure oder Arachinsäure ab. In einer bevorzugten Ausführungsform leitet sich die Gruppierung R¹-CO- von der Stearinsäure, der Palmitinsäure, der Ölsäure, einer Talgfettsäure oder einer Kokosfettsäure ab. Setzt man natürlich vorkommende Fettsäuren ein, kann es sich in vielen Fällen um Mischungen von Homologen und/oder um Mischungen von gesättigten und ungesättigten Fettsäuren gleicher Kohlenstoffatomzahl handeln (wie bei den genannten Talgfett- und Kokosfettsäuren).

Die Alkylengruppen A¹ und A² in den Aminoalkoholen II bezeichnen beispielsweise 1,1-Ethylen, 1,1-Propylen, 1,2-Propylen, 2,2-Propylen, 1,3-Propylen, 1,2-Butylen, 2,3-Butylen, 1,3-Butylen oder 1,4-Butylen, vor allem jedoch 1,2-Ethylen.

Stehen die Reste R² bis R⁶ für C₁- bis C₄-Alkyl, ist n-Propyl, iso-Propyl, n-Butyl, iso-Butyl, sec.-Butyl oder tert.-Butyl, vor allem jedoch Ethyl oder Methyl gemeint.

In einer bevorzugten Ausführungsform setzt man als Aminoalkohole solche der allgemeinen Formel IIa 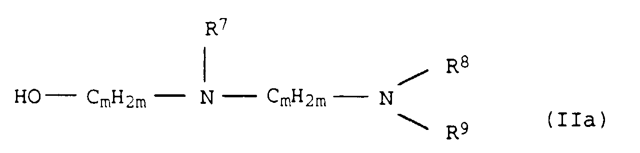 in der
- R⁷, R⁸ und R⁹: jeweils Wasserstoff, Methyl oder Ethyl bedeuten und
- m: für die Zahl 2 oder 3 steht.

Ganz besonders bevorzugt wird als Aminoalkohol N-(β-Aminoethyl)ethanolamin (R⁷=R⁸=R⁹=H, m=2) verwendet.

Die zur Umsetzung eingesetzten Aminoalkohole II können - insbesondere bei Einsatz technischer Qualitäten - in untergeordneten Mengen Mono-, Di- und/oder Trialkanolamine der Formel HO-A¹(bzw. A²)-NH₂, [HO-A¹(bzw. A²)]₂NH bzw. [HO-A¹(bzw. A²)]₃N enthalten, z.B. Mono-, Di- und/oder Triethanolamin. Um eine gute Emulgierbarkeit bzw. Wasserlöslichkeit der Kondensationsprodukte zu erreichen, sind maximal 20 Gew.-% der genannten Alkanolamine, insbesondere 10 Gew.-%, in den Aminoalkoholen II noch tolerabel. Bei Gehalten über 20 Gew.-% der genannten Alkanolamine wird die Wasserlöslichkeit der Kondensationsprodukte und somit die Emulgierbarkeit stark herabgesetzt. Man würde wäßrige Emulsionen erhalten, die in der üblichen Handelsform von mindestens 20 Gew.-% an Wirkstoffgehalt pastös und somit schwer zu handhaben wären.

In einer bevorzugten Ausführungsform setzt man als Polyole solche der allgemeinen Formel IIIa 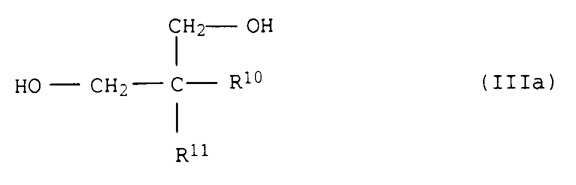 ein, in der
- R¹⁰: eine Hydroxylgruppe oder eine Hydroxymethylgruppe bezeichnet und
- R¹¹: Wasserstoff, Methyl, Ethyl oder eine Hydroxymethylgruppe bedeutet.

Ganz besonders bevorzugt werden als Polyole Glycerin (R¹⁰=OH, R¹¹=H), 1,1,1-Trimethylolethan und 1,1,1-Trimethylolpropan (R¹⁰=CH₂OH, R¹¹=CH₃ bzw. C₂H₅) sowie Pentaerythrit (R¹⁰=R¹¹=CH₂OH) verwendet.

Die erfindungsgemäßen Kondensationsprodukte werden zweckmäßigerweise dadurch hergestellt, daß man Fettsäuren I mit Aminoalkoholen II und Polyolen III in Gegenwart von katalytischen Mengen an phosphoriger Säure und/oder hypophosphoriger Säure erhitzt. Als katalytische Mengen können hier in der Regel 0,05 bis 2 Gew.-%, insbesondere 0,1 bis 1 Gew.-%, bezogen auf die Menge an eingesetzter Fettsäure I, angesehen werden.

Weiterhin führt man die Kondensation vorzugsweise bei Temperaturen von 150 bis 170°C durch. Das Reaktionswasser wird dabei ständig abdestilliert. Bei der Verwendung von phosphoriger Säure als Katalysator wird bei 160°C in etwa 4 Std. ein Umsetzungsgrad von >90 % erreicht. Das erhaltene Produkt wird gegebenenfalls mit Mineral- oder Carbonsäuren, beispielsweise mit Essigsäure, neutralisiert. Das erhaltene neutralisierte Wachs ist in Wasser selbstemulgierend und bei niedrigen Konzentrationen gut wasserlöslich und somit in der Handhabung bei der Anwendung problemlos.

Bei der beschriebenen Kondensation entstehen hauptsächlich Mischungen aus Fettsäurealkanolaminestern der allgemeinen Formel IV 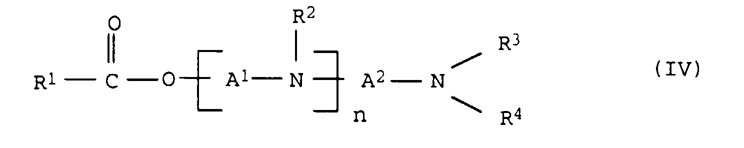 in der
- R¹: einen C₉- bis C₂₁-Alkyl- oder Alkenylrest bedeutet,
- R², R³ und R⁴: jeweils Wasserstoff, C₁- bis C₄-Alkyl oder eine Gruppierung -A¹-OH bzw. -A²-OH bedeuten,
- A¹ und A²: C₂- bis C₄-Alkylengruppen bezeichnen und
- n: für eine ganze Zahl von 1 bis 5 steht,
und Fettsäurehydroxyalkylestern der allgemeinen Formel V 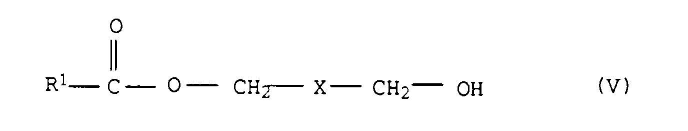 in der
- X: für eine chemische Bindung oder eine Gruppierung -CR⁵R⁶- steht, wobei
- R⁵: Wasserstoff, C₁- bis C₄-Alkyl, eine weitere Hydroxylgruppe oder eine Hydroxyalkylgruppe mit 1 bis 4 C-Atomen bezeichnet und
- R⁶: Wasserstoff, C₁- bis C₄-Alkyl oder eine Hydroxyalkylgruppe mit 1 bis 4 C-Atomen bedeutet,
im molaren Verhältnis IV:V von 5:95 bis 95:5.

Diese Mischungen sind ebenfalls Gegenstand der vorliegenden Erfindung.

Die erfindungsgemäßen Kondensationsprodukte und auch die erfindungsgemäßen Mischungen eignen sich in hervorragender Weise als Textilhilfsmittel, insbesondere als Behandlungsmittel zur Verminderung oder gar vollständigen Unterdrückung der Vergilbung beim Lagern von gefärbten Textilien, insbesondere von mit Indigo oder mit Indigo verwandten Farbstoffen gefärbten Textilien. Unter mit Indigo verwandten Farbstoffen sind Derivate des Indigo wie 6,6'-Dibromindigo, 5,5',7,7'-Tetrabromindigo, Indigocarmin, 4,4',7,7'-Tetrachlorindigo, Thioindigo, Methyl- und Chlor-Derivate des Thioindigo oder Indigorot zu verstehen. Im Prinzip können mit den erfindungsgemaßen Kondensationsprodukten bzw. Mischungen alle möglichen Textilfärbungen geschützt werden, beispielsweise Küpenfärbungen oder Färbungen mit Dispersionsfarbstoffen. Die erfindungsgemäßen Kondensationsprodukte bzw. Mischungen werden auf die zu schützenden Textilien normalerweise nach dem Färben appliziert.

Die erfindungsgemäßen Kondensationsprodukte bzw. Mischungen sind üblicherweise im Wasser selbstemulgierbar und haben einen kationischen Charakter. Diese kationische Ionogenität ist wichtig für die Anwendung nach dem Ausziehverfahren z.B. in Verbindung mit einer Wäsche oder einer Stonewäsche der Kleidungsstücke, die meist in den Industrie-Trommel-Waschmaschinen bei ca. 40°C vorgenommen wird. Beim Ausziehverfahren werden die erfindungsgemäßen Kondensationsprodukte bzw. Mischungen in einer Menge von 0,05 bis 10 Gew.-%, vorzugsweise 0,2 bis 4 Gew.-%, insbesondere 0,5 bis 2 Gew.-%, bezogen auf das Gewicht der Kleidungsstücke, dem Waschbad zugegeben. Durch das sehr gute Aufziehvermögen der erfindungsgemäßen Kondensationsprodukte bzw. Mischungen ziehen diese während des Waschvorganges vollständig auf das Textilgut auf.

Diese Applikationsmethode kommt den Denim-Wäschereien sehr entgegen. Die vergilbungsvermindernden Mittel können aber auch nach anderen Verfahren auf das Textilmaterial aufgebracht werden, z.B. durch Aufsprühen oder Foulardieren (Tauchen und Abquetschen). Das Foulardieren kommt jedoch nur bei Stückware in Betracht. Die aufgetragene Produktmenge ist die gleiche wie beim Ausziehverfahren.

Neben den vergilbungsvermindernden oder -hemmenden Eigenschaften verleihen die erfindungsgemäßen Kondensationsprodukte bzw. Mischungen dem Textilgut auch einen weichen Warengriff. Die weichmachende Wirkung läßt sich weiterhin bei der Ausrüstung von Textilien ausnutzen. Demgemäß eignen sich die erfindungsgemäßen Kondensationsprodukte bzw. Mischungen auch in hervorragender Weise als Weichmacher für die vergilbungsarme Hochveredlung von Textilien, wie sie beispielsweise in der eingangs zitierten EP-B 415 279 beschrieben ist.

Einzelheiten der Herstellung der erfindungsgemäßen Kondensationsprodukte und ihrer Anwendung können den nachfolgenden Beispielen entnommen werden, ohne jedoch den Umfang der vorliegenden Erfindung einzuschränken. Angaben über Teile und Prozente beziehen sich, sofern nicht anders vermerkt, auf das Gewicht.

### Herstellungsbeispiele

### Beispiel 1

Während der Umsetzung wurde ständig gerührt. Über das Reaktionsgemisch wurde Stickstoff geleitet. Das während der Umsetzung gebildete Reaktionswasser wurde ausgekreist (abdestilliert).

54 g 1,1,1-Trimethylolpropan und 217 g technische Stearinsäure (Stearinsäure/Palmitinsäure = 70 % / 30 %, Molgew. 284) wurden bei 80 bis 90°C im Glaskolben mit Stickstoffdurchlauf und Wasserbrücke aufgeschmolzen, anschließend wurden 42 g N-(β-Aminoethyl)ethanolamin sowie 0,65 g 50 %ige H3PO3 als Katalysator zugegeben.

Das Molverhältnis Fettsäure : Aminoethylethanolamin : Trimethylolpropan betrug 1,9 : 1 : 1. Die Katalysatormenge betrug 0,3 %, bezogen auf die Menge der verwendeten Fettsäure.

Das klare, gelbliche Reaktionsgemisch wurde auf 160°C erhitzt und bei dieser Temperatur 4,5 Std. reagieren gelassen.

In dieser Zeit erreichte die Säurezahl (potentiometrisch gemessen) einen Wert von 20 mg KOH/g, was einem Umsetzungsgrad von ca. 90 % entspricht.

Das flüssige Wachs wurde danach auf 80 bis 90°C abgekühlt und bei dieser Temperatur mit 26 g Essigsäure (99 %ig) neutralisiert.

### Herstellung der Emulsion:

80 g ca. 80°C heißes Wasser wurden vorgelegt, 20 g des Kondensationsproduktes als Schmelze zugesetzt und von selbst unter mäßigem Rühren auf Raumtemperatur abgekühlt. Man erhielt eine 20 %ige dünnflüssige Emulsion, die in den üblichen Anwendungsmengen klare, wäßrige Lösungen ergab.

### Beispiel 2

Es wurde in gleicher Weise wie im Beispiel 1 verfahren, jedoch mit dem Unterschied, daß anstelle von 1,1,1-Trimethylolpropan Glycerin verwendet wurde.

| | | |
|---|---|---|
| Einsatzmengen | Stearinsäure (technisch) | 217 g |
| | N-(β-Aminoethyl)ethanolamin | 42 g |
| | Glycerin(99,5 %ig) | 55 g |
| | H₃PO₃ (50 %ig) | 0,65 g |

Das Molverhältnis Fettsäure : Aminoethylethanolamin : Glycerin betrug 1,9 : 1 : 1,5.

Nach 4,5 Std. wurde eine Säurezahl von 19 mg KOH/g erreicht, was einem Umsetzungsgrad von ca. 92 % entspricht.

Eine wäßrige Emulsion wurde analog wie im Beispiel 1 hergestellt. Sie hatte die gleichen Merkmale wie diese.

### Beispiel 3

Es wurde in gleicher Weise wie im Beispiel 2 verfahren, jedoch mit dem Unterschied, daß neben N-(β-Aminoethyl)ethanolamin zusätzlich Diethanolamin mitverwendet wurde.

### Einsatzmengen:

| | | |
|---|---|---|
| Einsatzmenge | Stearinsäure (technisch) | 217 g |
| | N-(β-Aminoethyl)ethanolamin | 38 g |
| | Diethanolamin | 4 g |
| | Glycerin (99,5 %ig) | 55 g |
| | H₃PO₃ (50 %ig) | 0,65 g |

Das Molverhältnis Fettsäure : Aminoethylethanolamin : Diethanolamin : Glycerin betrug 1,9 : 0,9 : 0,1 : 1,5.

Nach 5 Std. wurde eine Säurezahl von 20 mg KOH/g erreicht, was einem Umsetzungsgrad von ca. 90 % entspricht.

Aus dem Kondensationsprodukt gemäß Beispiel 3 wurde eine wäßrige Emulsion wie im Beispiel 1 hergestellt.

Die Emulsion, hergestellt nach Beispiel 3, war bereits etwas dickflüssiger als die Emulsionen, die nach Beispiel 1 und 2 hergestellt wurden. Sie war auch in den Anwendungsmengen schlechter wasserlöslich, konnte jedoch noch problemlos angewandt werden.

### Anwendungsbeispiele

### Beispiel 4

Ein mit Indigo gefärbtes Gewebe (Jeansstoff) aus 100 % Baumwolle von etwa 150 g/m² (Prüflingsgröße ca. 10x20 cm) wurde in je einem Bad, das
(a) 5 % der Emulsion gemäß Beispiel 1
(b) 5 % der Emulsion gemäß Beispiel 2
(c) 5 % der Emulsion gemäß Beispiel 3
jeweils bezogen auf das Gewicht des Gewebes, enthielt, 15 min bei ca. 40°C behandelt, auf eine Badaufnahme von 80 % geschleudert und bei 100°C getrocknet.

Die behandelten Prüflinge sowie zum Vergleich ein unbehandelter wurden mit einem durchsichtigen Klebestreifen von ca. 2,5 cm Breite überklebt und eine Woche einer starken Luftzirkulation bei Raumtemperatur ausgesetzt. Die Prüflinge wurden visuell bewertet. Auf den durch die Streifen nicht geschützten Stellen zeigte das unbehandelte Gewebe deutliche Vergilbung der Indigofärbung. Bei 5 den behandelten Prüflingen konnte jedoch eine Vergilbung nicht erkannt werden. Die Prüflinge erhielten außerdem einen weichen, angenehmen Warengriff.

### Beispiel 5

Das gleiche Gewebe, das in Beispiel 4 verwendet worden war, wurde in ein Bad getaucht, welches jeweils 3 % der Emulsionen aus den Beispielen 1 bis 3 enthielt, anschließend auf dem Foulard auf eine Flottenaufnahme von etwa 80 % abgequetscht und bei ca. 100°C 5 getrocknet.

Die Prüflinge wurden wie im Beispiel 4 geprüft und visuell mit dem gleichen Ergebnis wie in Beispiel 4 bewertet.

## Patentansprüche

1. Kondensationsprodukte von Fettsäuren der allgemeinen Formel I 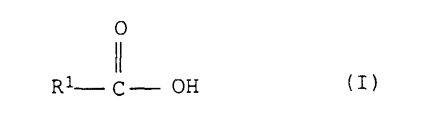 in der
R¹ einen C₉- bis C₂₁-Alkyl- oder -Alkenylrest bedeutet,
mit 5 bis 150 mol-% pro Mol I Aminoalkoholen der allgemeinen Formel II 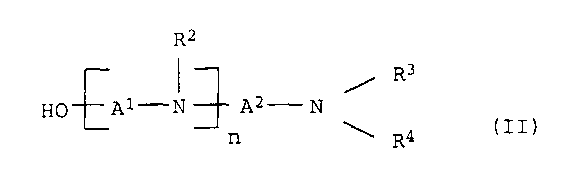 in der
A¹ und A² C₂- bis C₄-Alkylengruppen bezeichnen,
R², R³ und R⁴ jeweils Wasserstoff, C₁- bis C₄-Alkyl oder eine Gruppierung -A¹-OH bzw. -A²-OH bedeuten und
n für eine ganze Zahl von 1 bis 5 steht,
und 5 bis 200 mol-% pro Mol I Polyolen der allgemeinen Formel III
HO―CH₂―X―CH₂―OH (III)
in der
X für eine chemische Bindung oder eine Gruppierung -CR⁵R⁶- steht, wobei
R⁵ Wasserstoff, C₁- bis C₄-Alkyl, eine weitere Hydroxylgruppe oder eine Hydroxyalkylgruppe mit 1 bis 4 C-Atomen bezeichnet und
R⁶ Wasserstoff, C₁- bis C₄-Alkyl oder eine Hydroxyalkylgruppe mit 1 bis 4 C-Atomen bedeutet.

2. Kondensationsprodukte nach Anspruch 1 von Fettsäuren I mit 40 bis 70 mol-% pro Mol I Aminoalkoholen II und 30 bis 120 mol-% pro Mol I Polyolen III.

3. Kondensationsprodukte nach Anspruch 1 oder 2 von Fettsäuren I, bei denen sich die Gruppierung R¹-CO- von der Stearinsäure, der Palmitinsäure, der Ölsäure, einer Talgfettsäure oder einer Kokosfettsäure ableitet.

4. Kondensationsprodukte I nach den Ansprüchen 1 bis 3 mit Aminoalkoholen der allgemeinen Formel IIa 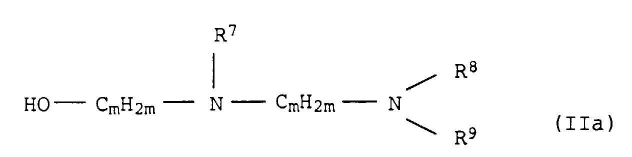 in der
R⁷, R⁸ und R⁹ jeweils Wasserstoff, Methyl oder Ethyl bedeuten und
m für die Zahl 2 oder 3 steht.

5. Kondensationsprodukte I nach den Ansprüchen 1 bis 4 mit Polyolen der allgemeinen Formel IIIa 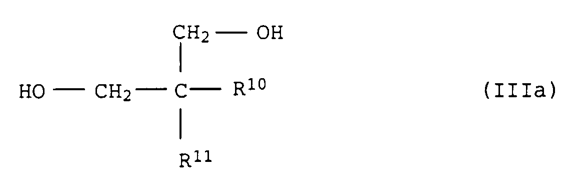 in der
R¹⁰ eine Hydroxylgruppe oder eine Hydroxymethylgruppe bezeichnet und
R¹¹ Wasserstoff, Methyl, Ethyl oder eine Hydroxymethylgruppe bedeutet.

6. Verfahren zur Herstellung von Kondensationsprodukten gemäß den Ansprüchen 1 bis 5, dadurch gekennzeichnet, daß man Fettsäuren I mit Aminoalkoholen II und Polyolen III in Gegenwart von katalytischen Mengen an phosphoriger Säure und/oder hypophosphoriger Säure erhitzt.

7. Verfahren zur Herstellung von Kondensationsprodukten nach Anspruch 6, dadurch gekennzeichnet, daß man die Kondensation der Fettsäuren I mit den Aminoalkoholen II und den Polyolen III bei Temperaturen von 150 bis 170°C durchführt.

8. Mischungen aus Fettsäurealkanolaminestern der allgemeinen Formel IV 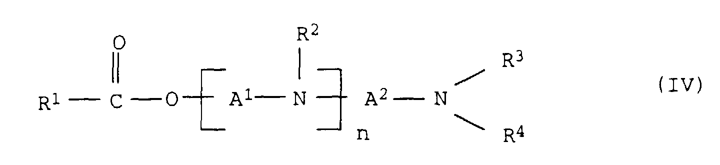 in der
R¹ einen C₉- bis C₂₁-Alkyl- oder Alkenylrest bedeutet,
R², R³ und R⁴ jeweils Wasserstoff, C₁- bis C₄-Alkyl oder eine Gruppierung -A¹-OH bzw. -A²-OH bedeuten,
A¹ und A² C₂- bis C₄-Alkylengruppen bezeichnen und
n für eine ganze Zahl von 1 bis 5 steht,
und Fettsäurehydroxyalkylestern der allgemeinen Formel V 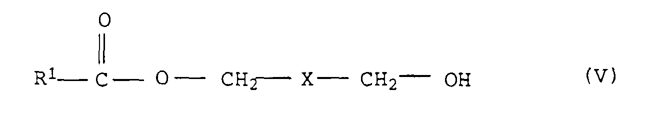 in der
X für eine chemische Bindung oder eine Grup pierung -CR⁵R⁶- steht, wobei
R⁵ Wasserstoff, C₁- bis C₄-Alkyl, eine weitere Hydroxylgruppe oder eine Hydroxyalkylgruppe mit 1 bis 4 C-Atomen bezeichnet und
R⁶ Wasserstoff, C₁- bis C₄-Alkyl oder eine Hydroxyalkylgruppe mit 1 bis 4 C-Atomen bedeutet,
im molaren Verhältnis IV:V von 5:95 bis 95:5.

9. Verwendung von Kondensationsprodukten gemäß den Ansprüchen 1 bis 5 und von Mischungen gemäß Anspruch 8 als Textilhilfsmittel, insbesondere als Behandlungsmittel zur Verminderung der Vergilbung beim Lagern von gefärbten Textilien und als Weichmacher für die vergilbungsarme Hochveredlung von Textilien.

10. Verfahren zur Verminderung der Vergilbung beim Lagern von gefärbten Textilien, dadurch gekennzeichnet, daß man diese Textilien nach dem Färben mit Kondensationsprodukten gemäß den Ansprüchen 1 bis 5 oder mit Mischungen gemäß Anspruch 8 behandelt.

11. Verfahren zur vergilbungsarmen Hochveredlung von Textilien, dadurch gekennzeichnet, daß man hierbei Kondensationsprodukte gemäß den Ansprüchen 1 bis 5 oder Mischungen gemäß Anspruch 8 als Weichmacher verwendet.

## Claims

1. Condensation products of fatty acids of the general formula I 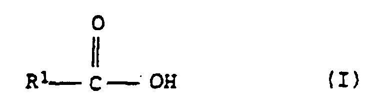 where R¹ is a C₉- to C₂₁-alkyl or -alkenyl radical, with from 5 to 150 mol% per mole of I of aminoalcohols of the general formula II 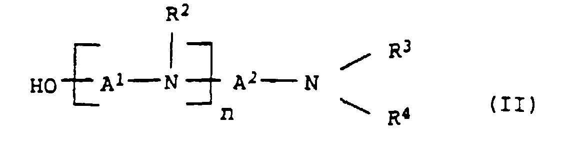 where A¹ and A² are C₂- to C₄-alkylene groups, R², R³ and R⁴ are each hydrogen, C₁- to C₄-alkyl or a -A¹-OH or -A²-OH grouping, and n is an integer from 1 to 5, and from 5 to 200 mol% per mole of I of polyols of the general formula III
HO―CH₂―X―CH₂―OH (III)
where X is a chemical bond or a -CR⁵R⁶- grouping, where R⁵ is hydrogen, C₁- to C₄-alkyl, a further hydroxyl group or a hydroxyalkyl group having from 1 to 4 carbon atoms, and R⁶ is hydrogen, C₁- to C₄-alkyl or a hydroxyalkyl group having from 1 to 4 carbon atoms.

2. Condensation products according to claim 1 of fatty acids I with from 40 to 70 mol% per mole of I of aminoalcohols II and from 30 to 120 mol% per mole of I of polyols III.

3. Condensation products according to claim 1 or 2 of fatty acids I, wherein the R¹-CO- grouping is derived from stearic acid, palmitic acid, oleic acid, a tallow fatty acid or a coconut fatty acid.

4. Condensation products I according to claims 1 to 3 with aminoalcohols of the general formula IIa 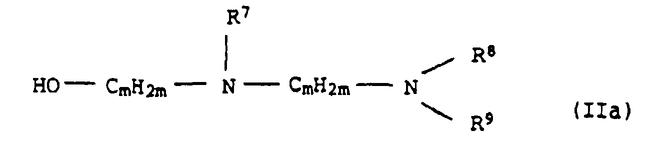 where R⁷, R⁸ and R⁹ are each hydrogen, methyl or ethyl, and m is 2 or 3.

5. Condensation products I according to claims 1 to 4 with polyols of the general formula IIIa 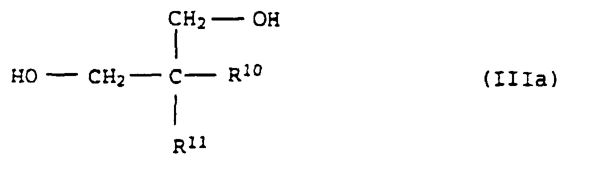 where R¹⁰ is a hydroxyl group or a hydroxymethyl group, and R¹¹ is hydrogen, methyl, ethyl or a hydroxymethyl group.

6. A process for preparing condensation products as claimed in claims 1 to 5, characterized in that fatty acids I are heated with aminoalcohols II and polyols III in the presence of catalytic amounts of phosphorous acid and/or hypophosphorous acid.

7. A process for preparing condensation products according to claim 6, characterized in that the condensation of the fatty acids I with the aminoalcohols II and the polyols III is carried out at temperatures of from 150 to 170°C.

8. Mixtures of fatty acid alkanolamine esters of the general formula IV 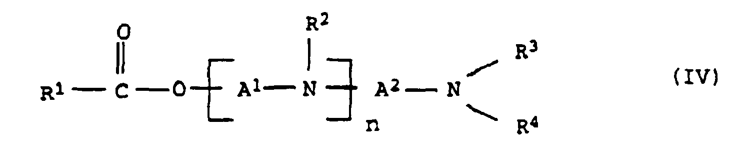 where R¹ is a C₉- to C₂₁-alkyl or -alkenyl radical, R², R³ and R⁴ are each hydrogen, C₁- to C₄-alkyl or a -A¹-OH or -A²-OH grouping, A¹ and A² are C₂- to C₄-alkylene groups, and n is an integer from 1 to 5,
and fatty acid hydroxyalkyl esters of the general formula V 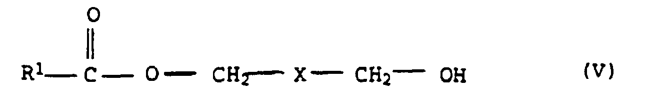 where X is a chemical bond or a -CR⁵R⁶- grouping, where R⁵ is hydrogen, C₁- to C₄-alkyl, a further hydroxyl group or a hydroxyalkyl group having from 1 to 4 carbon atoms, and R⁶ is hydrogen, C₁- to C₄-alkyl or a hydroxyalkyl group having from 1 to 4 carbon atoms, in a molar ratio of IV:V of from 5:95 to 95:5.

9. The use of condensation products as claimed in claims 1 to 5 and of mixtures as claimed in claim 8 as textile assistants, especially as treatment agents to reduce yellowing in storage of dyed textiles and as softeners for the low-yellowing resin finishing of textiles.

10. A process for reducing the yellowing in storage of dyed textiles, characterized in that these textiles are treated with condensation products as claimed in claims 1 to 5 or with mixtures as claimed in claim 8 after dyeing.

11. A process for the low-yellowing resin finishing of textiles, characterized in that condensation products as claimed in claims 1 to 5 or mixtures as claimed in claim 8 are used as softeners.

## Revendications

1. Produits de condensation d'acides gras de formule générale I 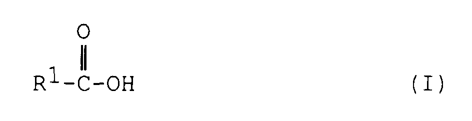 dans laquelle
R¹ représente un résidu alkyle ou alcényle en C₉ à C₂₁, avec 5 à 150% en moles, par mole de I, d'aminoalcools de formule générale II 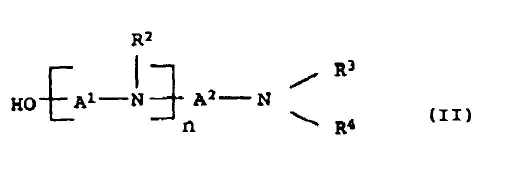 dans laquelle
A¹ et A² désignent des groupes alkylène en C₂ à C₄,
R², R³ et R⁴ représentent chacun un atome d'hydrogène, un groupe alkyle en C₁ à C₄ ou un groupement -A¹-OH ou bien -A²-OH et
n est mis pour un nombre entier de 1 à 5,
et 5 à 200% en moles, par mole de I, de polyols de formule générale III
HO-CH₂-X-CH₂-OH (III)
dans laquelle
X est mis pour une liaison chimique ou un groupement -CR⁵R⁶-,
R⁵ représentant un atome d'hydrogène, un groupe alkyle en C₁ à C₄, un groupe hydroxyle supplémentaire ou un groupe hydroxyalkyle ayant 1 à 4 atomes de carbone et
R⁶ représentant un atome d'hydrogène, un groupe alkyle en C₁ à C₄ ou un groupe hydroxyalkyle ayant 1 à 4 atomes de C.

2. Produits de condensation selon la revendication 1, d'acides gras I avec 40 à 70% en moles d'aminoalcools II par mole de I, et 30 à 120% en moles de polyols III par mole de I.

3. Produits de condensation selon la revendication 1 ou 2, d'acides gras I, dans lesquels le groupement R¹-CO- est dérivé de l'acide stéarique, de l'acide palmitique, de l'acide oléique, d'un acide gras de suif ou d'un acide gras de coprah.

4. Produits de condensation I selon les revendications 1 à 3, avec des aminoalcools de formule générale IIa 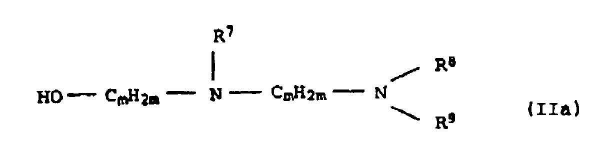 dans laquelle
R⁷, R⁸ et R⁹ représentent chacun un atome d'hydrogène, un groupe méthyle ou éthyle et
m est mis pour 2 ou 3.

5. Produits de condensation I selon les revendications 1 à 4, avec des polyols de formule générale IIIa 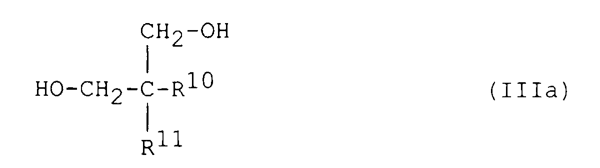 dans laquelle
R¹⁰ représente un groupe hydroxyle ou un groupe hydroxyméthyle et
R¹¹ représente un atome d'hydrogène, un groupe méthyle, éthyle ou un groupe hydroxyméthyle.

6. Procédé de préparation de produits de condensation selon les revendications 1 à 5, caractérisé en ce que l'on chauffe des acides gras I avec des aminoalcools II et des polyols III en présence de quantités catalytiques d'acide phosphoreux et/ou d'acide hypophosphoreux.

7. Procédé de préparation de produits de condensation selon la revendication 6, caractérisé en ce que l'on réalise la condensation des acides gras I avec les aminoalcools II et les polyols III à des températures de 150 à 170°C.

8. Mélanges d'esters d'alcanolamine et d'acide gras, de formule générale IV 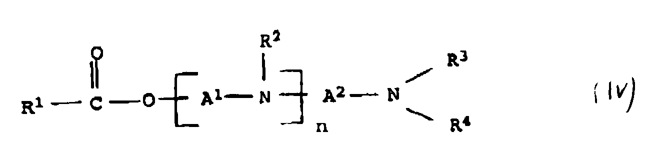 dans laquelle
R¹ représente un résidu alkyle ou alcényle en C₉ à C₂₁,
R², R³ et R⁴ représentent chacun un atome d'hydrogène, un groupe alkyle en C₁ à C₄ ou un groupement -A¹-OH ou bien -A²-OH,
A¹ et A² désignent des groupes alkylène en C₂ à C₄ et
n est mis pour un nombre entier de 1 à 5,
et d'esters hydroxyalkyliques d'acide gras de formule générale V 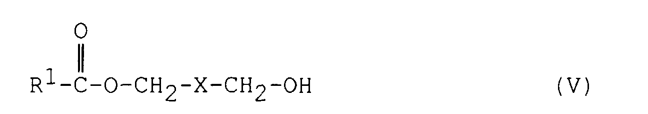 dans laquelle
X représente une liaison chimique ou un groupement -CR⁵R⁶-, où
R⁵ représente un atome d'hydrogène, un groupe alkyle en C₁ à C₄, un groupe hydroxyle supplémentaire ou un groupe hydroxyalkyle ayant 1 à 4 atomes de C et
R⁶ représente un atome d'hydrogène, un groupe alkyle en C₁ à C₄ ou un groupe hydroxyalkyle ayant 1 à 4 atomes de C,
dans le rapport molaire IV:V de 5:95 à 95:5.

9. Utilisation de produits de condensation selon les revendications 1 à 5 et de mélanges selon la revendication 8, en tant qu'adjuvants pour les textiles, en particulier comme agents de traitement pour diminuer le jaunissement lors de l'entreposage de textiles teintés et comme adoucissants pour l'ennoblissement à faible jaunissement, des textiles.

10. Procédé pour diminuer le jaunissement lors de l'entreposage de textiles teintés, caractérisé en ce que l'on traite ces textiles après teinture avec des produits de condensation selon les revendications 1 à 5 ou avec des mélanges selon la revendication 8.

11. Procédé pour l'ennoblissement à faible jaunissement, des textiles, caractérisé en ce que l'on utilise des produits de condensation selon les revendications 1 à 5 ou des mélanges selon la revendication 8 en tant qu'adoucissants.
